# EUROPEAN PATENT SPECIFICATION

(11) **EP 1 256 323 B1**
(45) Date of publication and mention of the grant of the patent: **09.08.2006**
(21) Application number: 02253131.3
(22) Date of filing: 03.05.2002
(51) Int. Cl.: A61B 17/32

(54) **Easily detachable ultrasonic clamping device**
Einfach abnehmbare Ultraschallklemme
Appareil ultrasonore à pince facilement démontable

(30) Priority: 04.05.2001 US 849905
(43) Date of publication of application: 13.11.2002
(73) Proprietor: ETHICON ENDO-SURGERY, Cincinnati, Ohio 45242 (US)
(72) Inventor: Witt, David Alan, Maineville, OH 45039 (US); Faller, Craig N., Milford, OH 45150 (US); Messerly, Jeffrey David, Cincinnati, OH 45209 (US); Baxter, Chester O., III, Loveland, OH 45140 (US); Schwemberger, Richard F., Cincinnati, OH 45247 (US); Neuenfeldt, Steven K., Cincinnati, OH 45249 (US); Houser, Kevin Lee, Springboro, OH 45066 (US); Haibel, Chester G., Jr., Mason, OH 45040 (US)
(74) Representative: Fisher, Adrian John

(56) References cited:
- WO-A-01/24713
- DE-A- 19 534 618
- US-A- 6 024 750
- US-B1- 6 214 023

## Description

### BACKGROUND OF THE INVENTION

### 1. Field of the Invention

The present invention relates to an ultrasonic surgical device which incorporates a readily detachable ultrasonic clamping device. In particular, the invention is directed to the provision of a removable blade tip and clamp assembly for ultrasonic treatments, so as to impart to the surgical device the ability of providing a variety of essentially exchangeable tips while reducing operationally engengered vibrations and stresses at the point of the coupling thereof to the clamp arm.

Ultrasonic surgical devices or instruments which include ultrasonically-operating shears require the employment of a clamping mechanism which clamps tissue between an ultrasonic blade and a clamp arm. The mechanism for actuating the clamping mechanism necessitates the installation of a movable inner tube which is adapted to activate the clamp arm, and a stationary tube on which the clamp arm pivots. These tubes considerably increase the expense of the surgical instrument, the cost of which is passed on to the customer or medical practitioner, and ultimately to the patient. In contrast, the inventive surgical instrument is designed to eliminate unnecessary costly components while being capable of reducing the outer diameter of the instrument or device. There is also a need in the medical-technology for an ultrasonic instrument which possesses a detachable tip structure in order to facilitate cleaning, disposal, or usage of various end effectors; such as the blade and clamp element. A problem is also encountered with current detachable tip instruments in that they have an excessive number of parts, rendering the tips cumbersome to construct, expensive to manufacture; and also difficult to assemble by various possibly semi-skilled personnel and medical practitioners.

Although the medical technology is extensively concerned with the problems encountered in connection with the construction of ultrasonic clamping devices or surgical instruments of generally the type considered herein, various desirable constructive and functional aspects are clearly lacking in the current state-of-the-technology.

### 2. Discussion of the Prior Art

Terumasa Japanese unexamined patent application 9-38099 discloses an ultrasonic surgical instrument with a tip/clamp assembly located at the distal node of a blade extender. The clamp arm of the instrument is movable on an axial pin, whereas the tip end is detachable through the use of a threaded screw connection, whereby this connection facilitates the interchanging of the tips. Due to the housing being located at the node, the point of attachment for the interchangeable tips is not located at the node, whereby the clamp assembly is not connected directly to the waveguide, but rather is attached to a housing located at the distal node of the blade extender.

Mitsumasa Japanese unexamined patent application 8-275952 discloses an ultrasonic surgical tool with a blade/clamp assembly which is affixed to the blade via a nodally mounted block. The clamp is held open by a resilient flexible material; whereas the outer tube may then be actuated over the clamp through the intermediary of a trigger assembly for effectively closing the clamp which becomes ensheathed by the outer tube. The tip/clamp assembly may also be removed for cleaning/disposal by means of a threaded connection.

Schad U.S. Patent No. 5,676,678 is directed to the provision of a surgical instrument with a holder for various tips, wherein the holder is coupled to an inner tube, and with the holder being detachable from the inner tube of the surgical instrument. The coupling functions by using two snap legs which engage over the tip assembly to fix the holder; the snap legs possessing elastic qualities which facilitate the selective attachment and removal of the tip assembly. When the holder coupling is exposed, the outer tube is held in place against the pressure of a helical spring by engaging hook projections located in an undercut rim of the instrument. These projections may then be released to cover and protect the coupling.

Schad German Patent 19 534 618 issued on March 20, 1997 discloses a surgical instrument with jaw components whereby at least one jaw part is connected to an inner tube so that it can be actuated relative to an outer tube to which the jaws are pivotally mounted. The jaw part consists of a catch which inserts into a bore hole opening, and closing the jaw part is effected by rotating the latter around a transversely mounted pin connecting the jaw parts to the outer tube. The instrument is designed to be disassembled through the provision of a removable inner tube so as to facilitate cleaning or disposal thereof. This patented surgical instrument is designed for manual and electrical use rather than as an ultrasonic surgical device. In contrast, the present invention possesses a clamp/blade system which is more effective in ultrasonic devices.

Hood U.S. Patent No. 5,669,922 discloses an ultrasonic surgical instrument having an end assembly comprising an end hook which is threaded and screwed into complementary threads formed on or in an extender. The blade of the instrument has a step that is located at or proximate the nodal location of the assembly. This patent discloses only a blade, whereas to the contrary, the present invention incorporates a clamp mechanism, and moreover locates the coupling of the clamp mechanism at the node. By locating the coupling of the clamp at the node, mechanism pursuant to the present invention, vibrations and consequently stresses acting on the coupling are reduced. The nodal placement of the coupling functions to decrease wear on the coupling and reduces the possibility of clamp arm/transverse mounted pin/blade movement due to ultrasonic vibrations.

Davidson et al. Patent No. 5,322,055 discloses an ultrasonic surgical apparatus comprising a housing, an ultrasonic element carried by the housing for generating ultrasonic vibration, a blade coupled to the ultrasonic element and a clamp which moves in opposition to the blade for use in clamped coagulation. The present invention patentably distinguishes thereover by including a removable tip area which is located in an anti-nodal region, a multitude of choices for the tube/lever closure of the clamping device, a disassembly capability and a construction possessing fewer parts.

DiMatteo, et al U.S. Patent NO. 5,810,859 discloses an ultrasonic surgical instrument with a removable waveguide which is connected to the hub of an ultrasonic transducer handle. The hub is designed to enable a coupling member to apply a rotational torque to the hub of the outer sheath, which is to be transmitted to the waveguide in order to tighten it onto the mounting device of the handpiece assembly. In a preferred embodiment of the patent, the location of the connection of the waveguide and handpiece is at the node to reduce any wear and stress due to the ultrasonic vibrations. In contrast, the present invention features a nodal connection at the point of attachment of the movable end effector; whereas although the patent discloses an embodiment with a removable end effector, that embodiment does not provide any disclosure about the nodal location of the movable end effector (jaw) coupling. The nodal location of the movable end effector pursuant to the present invention, in contrast, reduces wear on the coupling and reduces the risk of the tip attachment loosening or becoming damaged and the blade becoming damaged due to ultrasonic vibrations.

Mastri et al. U.S. Patent No. 6,024,750 discloses an ultrasonic dissection and coagulation system including an ultrasonic instrument with a housing, an elongated body portion extending from the housing and a cutting jaw. A clamp member having a tissue contact surface is positioned adjacent to the cutting jaw and is movable from an open position, to a clamped position in which tissue is clamped between the cutting jaw and the tissue contact surface.

### SUMMARY OF THE INVENTION

Accordingly, in order to overcome the drawbacks and limitations encountered in the prior art, the present invention discloses an ultrasonic surgical instrument comprising:
an end effector including relatively movable blade and clamp means for the engagement of tissues located therebetween; an elongated shaft element having said end effector arranged at a first end thereof;
an elongated tubular member extending about said elongated shaft element in coaxial relationship, said elongated tubular member having a first end in operative engagement with said end effector;
a handle portion for receiving second opposite ends of respectively said elongated shaft element and said elongated tubular member, said handle portion including finger-actuatable scissors-like trigger means for imparting axial displacement between said elongated shaft element and said elongated tubular member so as to cause said blade and clamp means to selective open and close relative to each other, characterised in that:
said elongated tubular member is fixedly attached to said handle portion, said trigger means having a pivotable portion hingedly connected to the second end of said elongated shaft element, whereby actuation of said pivotable trigger portion imparts said axial displacement to said elongated shaft element relative to said elongated tubular member.

Among the advantages of the present invention there may be included a thumb trigger/limiter assembly for controlling the amount of force applied to the instrument. A spring which may be embodied in the limiter assembly absorbs excessive force which may be possibly applied by the physician so as to prevent potential breakage of the instrument. The present invention also features an actuating blade assembly which actuates within an outer tube. This system eliminates the need for an inner tube, while at the same time eliminating the inadvertent friction caused by instruments in the prior art actuated by outer tubes which rub on the trocar. A detachable blade/tip/clamp assembly may allow for the utilization of easily cleanable, disposable, and quickly interchangeable tips. Though there are several embodiments of the latter, the present invention may also have these parts affixed to each other, thereby reducing the extra parts necessary to make an instrument capable of being disassembled. These fixed instruments are beneficial for use in surgical or medical procedures where an inexpensive, disposable instrument would be optimal in order to reduce healthcare expenditures. Possible location of the clamp arm at the node reduces vibration and stress due to ultrasonic vibrations, thereby reducing wear and the potential of any breakage of the clamp arm. The blade tip apparatus may be located at or near the antinode to transfer as much ultrasonic power as possible to the blade. A direct drive system which may connect the clamp arm to the trigger of the instrument serves to provide the physician with controlled forceful blunt dissection. In effect, the novel ultrasonic surgical instrument offers a physician a system adapted to apply a more controlled force in both opening and closing of the clamp arm.

Pursuant to a specific embodiments, the present invention uses a pin passing through the node and some method of grounding the clamp to the tube. Opening and closing could be facilitated through either blade movement relative to the tube or tube movement relative to the blade. Both movements would be initiated by the user via a trigger assembly; whereby this system eliminates the possibility that a loss of elasticity and control may occur over time as stress is applied to the resilient flexible material. The trigger control system of the present invention provides the surgeon, physician or other medical practitioner, such as a nurse with greater control and reliability. The present invention may also incorporate a direct drive system as opposed to the spring system of Mitsumasa Japanese '952. One embodiment of the present invention provides a screw-in connection that consists of a single contained piece that may be easily removed or attached. The screw-in connection provides for faster interchange or removal of tips and reduces overall part costs. However, other attachment methods may be utilized such as by means of magnaforming, press fit, swaging, and so forth.

Accordingly it is an object of the present invention to provide an improved ultrasonic surgical instrument which incorporates an easily detachable and replaceable ultrasonic clamping device.

Another object of the invention resides in the provision of a ultrasonic surgical instrument possessing a unique attaching structure for the clamping blade and tip device which reduces stress and vibrations encountered due to ultrasonic vibrations so as to extend the service life of the instrument.

Yet another object of the invention is to provide an ultrasonic instrument of the type described which employs a minimum number of easily assembled parts so as to render the instruments inexpensive to manufacture and render components of the instrument economically disposable and replaceable after only a single use.

### BRIEF DESCRIPTION OF THE DRAWINGS

Reference may now be made to the following detailed description of preferred embodiments of ultrasonic surgical instruments pursuant to the invention, taken in conjunction with the accompanying drawings; in which:
Figure 1 illustrates a longitudinal view, partly in section, of a first embodiment of the ultrasonic surgical instrument pursuant to the invention;
Figure 2 illustrates, in a view similar to that of Fig. 1, an alternative ultrasonic surgical instrument;
Figure 3 illustrates a detailed view of the extender arm and blade/clamp assembly of the instrument of Fig. 2;
Figure 4 illustrates a detailed view of an arrangement for attaching the blade/clamp tip portion of the instrument to an extender arm;
Figure 5 illustrates, on an enlarged scale, the clamp/blade tip portion of Fig. 4;
Figure 6 illustrates a further embodiment of the actuator handle portion of an ultrasonic surgical instrument; and
Figure 7 illustrates a diagrammatic spring limiter arrangement for minimizing stress on the clamp/blade tip assembly of the instrument of Fig. 6.

### DETAILED DESCRIPTION OF PREFERRED EMBODIMENTS

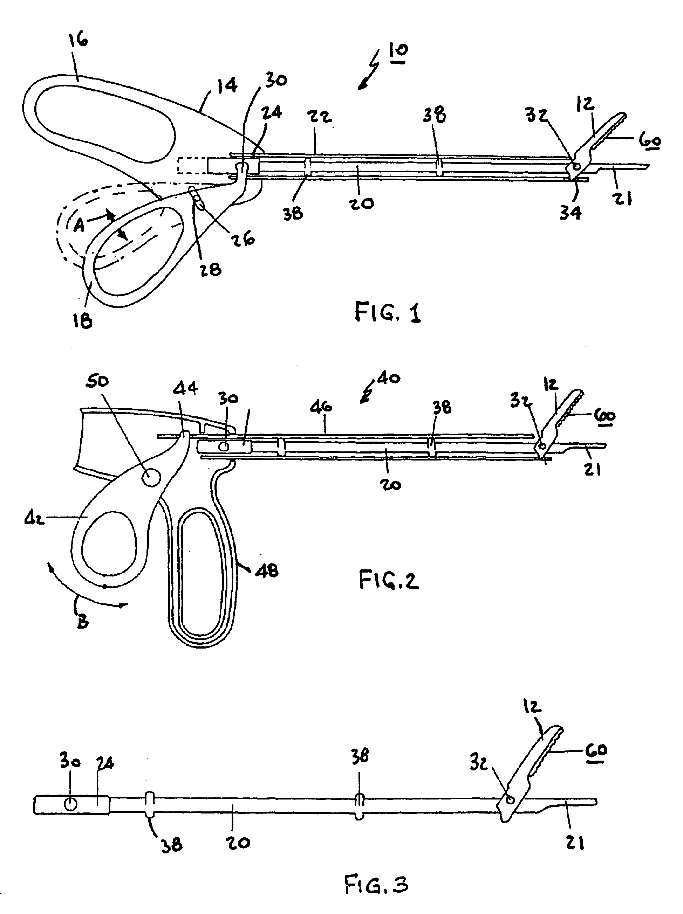

Referring in detail to the drawings, Figure 1 is a longitudinal sectional view of an ultrasonic surgical instrument 10 constructed pursuant to one embodiment. As shown, the ultrasonic surgical instrument 10 includes a coagulating accessory clamp arm 12. Ultrasonic surgical instrument 10 comprises a handle-forming housing structure 14 including scissor-like operable trigger portion 16, 18 actuatable by the thumb and finger of a user along arrow A. A blade extender 20 in the form of an elongated rod or shaft extends from the housing structure to an ultrasonic blade 21 which in this embodiment is integral therewith. A removable transducer (not shown) is located in the housing structure 14, and is preferably a piezeoceramic transducer for converting an electrical signal, for example, a 55,000 Hz sinusoidal waveform, into a mechanical longitudinal vibration. Handle structure 14 connects to an outer sheath or tube 22 coaxially covering the blade extender 20, with the tube 22 having an end 24 fixedly attached to the housing structure, with blade extender 20 being axially slideable relative to outer tube 22. In the embodiment of the ultrasonic surgical instrument 10 of Fig. 1, the trigger portion 18 is adapted to rotate around a series of cam surfaces 26 on a pivot boss 28 on handle 14, and is connected to the blade extender or shaft 20 by means of an insertion arm 30.

The clamp arm 12 connects directly in a hinged manner to the ultrasonic blade 21 through the intermediary of a pin 32 about which the arm 12 may rotate.
Though a pin 32 is used in this embodiment, there are numerous other means of hingedly connecting the clamp arm 12 to the ultrasonic blade 21, such as through a rivet, screw or the like. The clamp arm 12, which is attached to the ultrasonic blade 21, then protrudes through a series of holes 34 formed in the outer tube 22.

During operation, wherein the trigger portion 18 of the handle is pulled towards trigger portion 16 of handle part 14 in a finger actuated scissor-motion rotating around the cam surfaces of the pivot boss 28, whereby the insertion arm 30 which is connected to the blade extender 20 either mechanically or by some mechanical transfer device (not shown), actuates the blade extender 20 proximally so as to move axially within outer tube 22. By actuating the blade extender 20 proximally, the end of the clamp arm 12 which protrudes from a series of holes 34 is pulled backward against the wall of outer tube 22. As the proximal end of clamp arm 12 is pulled back, the clamp arm 12 rotates around pin 32, rotating clamp arm 12 closer to blade 21. When the trigger portion 18 is pulled away from the handle trigger portion 16, the insertion arm 30 pivots around the cam surfaces of the pivot boss 28, thereby actuating the blade extender 20. As the blade extender 20 moves distally, the proximal end of the clamp arm 12 which protrudes through the series of holes 34 is pushed distally by the outer tube 22. In doing so, the clamp arm 12 rotates around pin 32 rotating the distal end of the arm away from the blade.

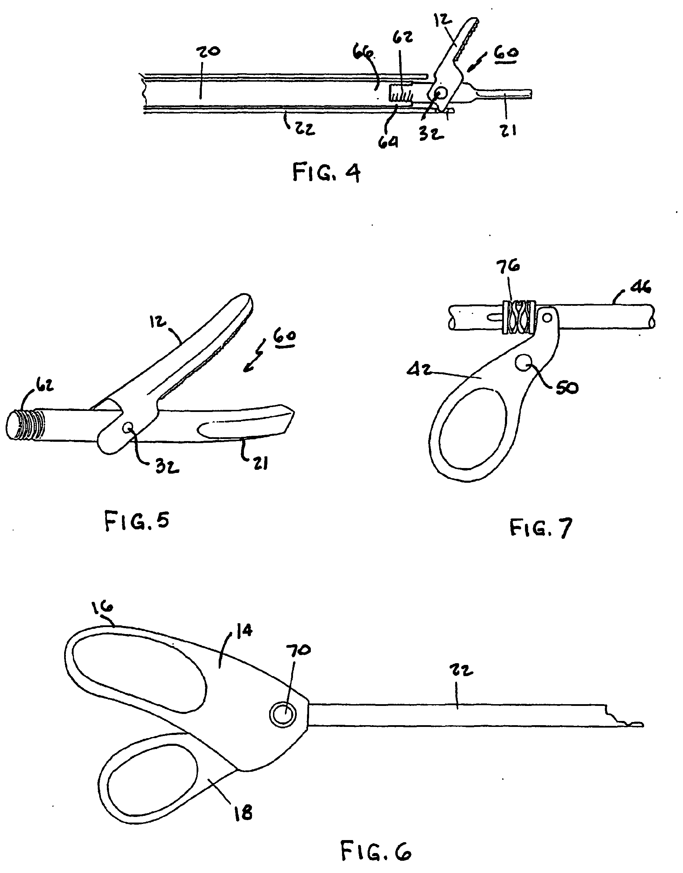

As shown in Fig. 3, rings 38 are formed spaced along the length of the blade extender 20 and ultrasonic blade 21 at the nodes thereof so as to prevent the dispersion of ultrasonic waves to the outer tube 22. It is also possible to provide a reverse of this functioning with regard to the way in which the clamp arm 12 opens and closes, depending upon actuating direction in the trigger of the handle mechanism. The ultrasonic surgical instrument 10 may be built with the trigger portion 16 as the stationary actuator or with trigger portion 18 as the actuator mechanism, or reversely.

In Figure 2, in which elements similar to those in Fig. 1 are identified by the same reference numerals, there is illustrated a second instrument in which a trigger 42 of the instrument 40 connects by means of an actuator attachment 44 to outer tube 46. The trigger 42 is connected to a handle 48 through a pivot boss 50 about which trigger 42 pivots. In operation, trigger 42 is pushed towards handle 48 rotating in directions of arrow B about the pivot boss 50. The actuator attachment 44 is connected to the outer tube 46 by projecting into a slot 52 such that when the latter pivots counterclockwise around pivot boss 50, pulling the outer tube 46 proximally. As the outer tube 46 moves at its further or distal end, clamp arm 12 is pushed towards blade 21 as it pivots around pin 32. When the trigger 42 is pulled away from handle 48, the actuator attachment 44 pivots proximally around the pivot boss 50, whereupon as the actuator attachment 44 pivots, and the outer tube 46 is also pulled distally. As the outer tube moves distally, it resultingly pulls against the proximal end of the clamp arm 12 protruding though a series of small holes 34. This produces the effect of rotating the clamp arm 12 around the pin 32 and pushing the clamp arm 12 closer to the blade 21.

As illustrated in Figure 4, the tip portion 60 of the ultrasonic surgical instrument 10 or 40, of either Figures 1 and 2, may be formed as a separate element from the blade extender arm or shaft 20. In this instance, the tip portion 60, which comprises the blade 21 pivotally connected by pin 32 to the clamp arm 12, as shown in detail in Figure 5, has the blade 21 equipped with a screw threaded end 62, which is adapted to threadingly engage a complimentary screwthread 64 formed in the end 66 of the blade extender arm 20. Other types of connections can also be provided, such by pin locks, interference fit, magnaforming or the like. This construction allows for easy removal and exchange of the tip portion 60, without requiring the entire instrument to be replaced.

Referring to the schematic representation of Figure 6, this shows the provision of a latching pushbutton 70 on the handle structure 14 of the ultrasonic surgical instrument 10 of Fig. 1, which is connected to the outer tube 22, which may normally prevent inadvertent actuation of the instrument. Depressing the pushbutton 70, which may be spring-loaded, releases trigger portions, enabling the functioning of the blade and clamp assembly by allowing the trigger portions 16 and 18 to pivot relative to each other as previously described.

In Figure 7, a spring limiter system 76 which is located on the outer tube 46 acts to minimize stress transmitted to the blade assembly 60 by absorbing any excessive force applied by the physician to trigger portions 16 or 18. The spring limiter system 76 is located near the proximal end of the ultrasonic surgical instrument 40 adjacent the handle structure 48.

While the invention has been particularly shown and described with respect to preferred embodiments thereof, it will be understood by those skilled in the art that the foregoing and other changes in form and details may be made therein without departing form the scope of the invention.

## Claims

1. An ultrasonic surgical instrument (10) comprising:
an end effector including relatively movable blade (21) and clamp means (12) for the engagement of tissues located therebetween;
an elongated shaft element (20) having said end effector arranged at a first end thereof;
an elongated tubular member (22) extending about said elongated shaft element (20) in coaxial relationship, said elongated tubular member (22) having a first end in operative engagement with said end effector;
a handle portion (14) for receiving second opposite ends of respectively said elongated shaft element (20) and said elongated tubular member (22), said handle portion (14) including finger-actuatable scissors-like trigger means (16, 18) for imparting axial displacement between said elongated shaft element (20) and said elongated tubular member (22) so as to cause said blade (21) and clamp means (12) to selective open and close relative to each other, **characterised in that**:
said elongated tubular member (22) is fixedly attached to said handle portion (14), said trigger means (16,18) having a pivotable portion (18) hingedly connected to the second end of said elongated shaft element (20) whereby actuation of said pivotable trigger portion (18) imparts said axial displacement to said elongated shaft element (20) relative to said elongated tubular member (22).

2. An ultrasonic surgical instrument (10) as claimed in Claim 1, wherein said pivotable trigger portion (18) is rotatable about slotted cam surface (26) on a pivot boss (28) fixed to a stationary part of said handle portion (14).

3. An ultrasonic surgical instrument (10) as claimed in Claim 2, wherein an insertion arm (30) on said pivotable trigger portion (18) is operatively engaged with said elongated shaft element (20) for imparting axial movement thereto responsive to rotation of said pivotable trigger portion (18) about said cam surfaces (26).

4. An ultrasonic surgical instrument (10) as claimed in any preceding claim, wherein said first ends of said elongated shaft element (20) and of said elongated tubular member (22) comprise cooperative camming structure for selectively opening and closing said blade (21) and clamp means (12) responsive to relative axial movement between said shaft element (20) and tubular member (22).

5. An ultrasonic surgical instrument (10) as claimed in any preceding claim, wherein the blade (21) of said end effector comprises a coaxial tip on said elongated shaft element (20).

6. An ultrasonic surgical instrument (10) as claimed in Claim 5, wherein said blade (21) comprises a stub shaft integrally formed at the first end of said elongated shaft element (20), whereby said shaft element (20) forms an ultrasonic blade extender.

7. An ultrasonic surgical instrument (10) as claimed in Claim 5, wherein said blade (21) comprises a stub shaft which is detachably fastened to the first end of said elongated shaft element (20), whereby said shaft element (20) forms an ultrasonic blade extender.

8. An ultrasonic surgical instrument (10) as claimed in Claim 7, wherein said blade (21) is fastened to said elongated shaft element (20) through a screw threaded connection (64).

9. An ultrasonic surgical instrument (10) as claimed in claim 4, wherein said caming structure comprises a cam arm mounted on said blade (21) for pivotal movement relative thereto.

10. An ultrasonic surgical instrument (10) as claimed in any preceding claim, wherein said handle portion (14) includes trigger-latching pushbutton means (70) for release of said trigger portions (16,18) from a latched condition responsive to depression of said pushbutton (70) so as to enable relative movement between said shaft element (20) and the tubular member (22) upon actuation of said trigger portion (18).

11. An ultrasonic surgical instrument (10) as claimed in any preceding claim, wherein a plurality of axially spaced rings (38) are formed at nodes along the length of said elongated shaft element (20) and blade (21) as to prevent dispersion of ultrasonic waves to said surrounding elongated tubular member (22) during operation of said instrument.

12. An ultrasonic surgical instrument (10) as claimed in any preceding claim, wherein spring limiter means (76) are formed on said elongated tubular member (22) so as to absorb excessive operating forces and stresses generated responsive to actuation of said handle portion (14).

## Patentansprüche

1. Mit Ultraschall arbeitendes chirurgisches Instrument (10), mit:
einem End-Effektor, welcher eine relativbewegbare Klinge (21) und eine Klemmeinrichtung (12) für das Greifen von dazwischen befindlichem Gewebe umfaßt;
einem länglichen Wellenelement (20), auf dessen ersten Ende der End-Effsktor angeordnet ist;
einem länglichen rohrförmigen Element (22), der sich um das längliche Wellenelement (20) in koaxialer Lagebeziehung erstreckt, wobei das längliche rohrförmige Element (22) ein erstes Ende in operativem Eingriff mit dem End-Effektor hat;
einen Griffabschnitt (14) zum Aufnehmen zweiter gegenüberliegender Enden jeweils des länglichen Wellenelementes (20) und des länglichen rohrförmigen Elementes (22), wobei der Griffabschnitt (14) fingerbetätigbare scherenartige Triggereinrichtungen (16, 18) umfaßt, um zwischen dem länglichen Schaftelement (20) und dem länglichen rohrförmigen Element (22) eine axiale Verlagerung aufzugeben, um so zu bewirken, daß die Klinge (21) und die Klemmeinrichtung (12) sich wahlweise relativ zueinander öffnen und schließen, **dadurch gekennzeichnet, daß**:
das längliche rohrförmige Element (22) fest an dem Griffabschnitt (14) befestigt ist, die Triggereinrichtung (16, 18) einen schwenkbaren Abschnitt (18) hat, der gelenkig an dem zweiten Ende des länglichen Wellenelementes (20) angebunden ist, wobei die Betätigung des schwenkbaren Triggerabschnittes (18) die axiale Verlagerung auf das längliche Wellenelement (20) relativ zu dem länglichen rohrförmigen Element (22) aufgibt.

2. Mit Ultraschall arbeitendes chirurgisches Instrument (10) nach Anspruch 1, bei dem die schwenkbare Triggereinrichtung (18) entlang einer schlitzartigen Kurvenfläche (26) auf einem Schwenkzapfen (28), der an einem stationären Teil des Griffabschnittes (14) befestigt ist, drehbar ist.

3. Mit Ultraschall arbeitendes chirurgisches Instrument (10) nach Anspruch 2, bei dem ein Einführarm (30) auf dem schwenkbaren Triggerabschnitt (18) operativ im Eingriff mit dem länglichen Wellenelement (20) zum Aufgeben der axialen Bewegung auf dieses in Antwort auf die Drehung des schwenkbaren Triggerabschnittes (18) entlang der Kurvenfläche (26) ist.

4. Mit Ultraschall arbeitendes chirurgisches Instrument (10) nach einem der vorangehenden Ansprüche, bei dem die ersten Enden des länglichen Wellenelementes (20) und des länglichen rohrförmigen Elementes (22) eine zusammenwirkende kämmende Struktur zum wahlweisen Öffnen und Schließen der Klinge (21) und der Klemmeinrichtung (12) in Antwort auf die relative axiale Bewegung zwischen dem Wellenelement (20) und dem rohrförmigen Element (22) aufweisen.

5. Mit Ultraschall arbeitendes chirurgisches Instrument (10) nach einem der vorangehenden Ansprüche, bei dem die Klinge (21) des End-Effektors eine koaxiale Spitze auf dem länglichen Wellenelement (20) aufweist.

6. Mit Ultraschall arbeitendes chirurgisches Instrument (10) nach Anspruch 5, bei dem die Klinge (21) einen Stummelschaft aufweist, der einstückig an dem ersten Ende des länglichen Wellenelementes (20) angebracht ist, wobei das Wellenelement (20) einen Ultraschall-Klingenextender bildet.

7. Mit Ultraschall arbeitendes chirurgisches Instrument (10) nach Anspruch 5, bei dem die Klinge (21) einen Stummelschaft aufweist, der lösbar an dem ersten Ende des länglichen Wellenelementes (20) angebracht ist, wobei das Wellenelement (20) einen Ultraschall-Klingenextender bildet.

8. Mit Ultraschall arbeitendes chirurgisches Instrument (10) nach Anspruch 7, bei dem die Klinge (21) an dem länglichen Wellenelement (20) durch eine Schraubgewindeverbindung (64) befestigt ist.

9. Mit Ultraschall arbeitendes chirurgisches Instrument (10) nach Anspruch 4, bei dem die kämmende Struktur einen Nockenarm, der auf der Klinge (21) für die Schwenkbewegung relativ zu dieser angeordnet ist, aufweist.

10. Mit Ultraschall arbeitendes chirurgisches Instrument (10) nach einem der vorangehenden Ansprüche, bei dem der Griffabschnitt (14) eine den Trigger lösende Druckknopfeinrichtung (70) zum Freigeben der Triggerabschnitte (16, 18) aus einem verriegelten Zustand in Antwort auf das Drücken des Druckknopfes (70) umfaßt, um so die relative Bewegung zwischen dem Wellenelement (20) und dem rohrförmigen Element (22) beim Betätigen des Triggerabschnittes (18) zu ermöglichen.

11. Mit Ultraschall arbeitendes chirurgisches Instrument (10) nach einem der vorangehenden Ansprüche, bei dem eine Vielzahl axial beabstandeter Ringe (38) an Knoten entlang der Länge des länglichen Wellenelementes (20) und der Klinge (21) gebildet sind, um die Streuung von Ultraschallwellen in das umgebende längliche rohrförmige Element (20) während des Betriebes des Instrumentes zu verhindern.

12. Mit Ultraschall arbeitendes chirurgisches Instrument (10) nach einem der vorangehenden Ansprüche, bei dem Federbegrenzereinrichtungen (76) auf dem länglichen rohrförmigen Element (22) gebildet sind, um so übermäßige Betriebskräfte und Belastungen zu absorbierenden, die in Antwort auf das Betätigen des Griffabschnitts (14) erzeugt werden.

## Revendications

1. Instrument chirurgical ultrasonore (10), comprenant :
■ un effecteur comprenant des moyens de lame (21) et de serrage (12) relativement mobiles pour la mise en prise des tissus situés entre eux ;
■ un élément de tige allongé (20) ayant ledit effecteur agencé au niveau de sa première extrémité ;
■ un élément tubulaire allongé (22) s'étendant autour dudit élément de tige allongé (20) selon une relation coaxiale, ledit élément tubulaire allongé (22) ayant une première extrémité en mise en prise opérationnelle avec ledit effecteur ;
■ une partie de poignée (14) pour recevoir les secondes extrémités opposées respectivement dudit élément de tige allongé (20) et dudit élément tubulaire allongé (22), ladite partie de poignée (14) comprenant des moyens de déclencheur (16, 18) en forme de ciseaux, pouvant être actionnés avec les doigts, pour communiquer le déplacement axial entre ledit élément de tige allongé (20) et ledit élément tubulaire allongé (22) afin d'amener lesdits moyens de lame (21) et de serrage (12) à s'ouvrir et à se fermer de manière sélective l'un par rapport à l'autre, **caractérisé en ce que**
■ ledit élément tubulaire allongé (22) est fixé de manière fixe sur ladite partie de poignée (14), lesdits moyens de déclencheur (16, 18) ayant une partie pivotante (18) raccordée par articulation à la seconde extrémité dudit élément de tige allongé (20), moyennant quoi l'actionnement de ladite partie de déclencheur (18) pivotante communique ledit déplacement axial audit élément de tige allongé (20) par rapport audit élément tubulaire allongé (22).

2. Instrument chirurgical ultrasonore (10) selon la revendication 1, dans lequel ladite partie de déclencheur (18) pivotante peut tourner autour de la surface de came fendue (26) sur une bosse de pivot (28) fixée sur une partie fixe de ladite partie de poignée (14).

3. Instrument chirurgical ultrasonore (10) selon la revendication 2, dans lequel un bras d'insertion (30) situé sur ladite partie de déclencheur (18) pivotante est mis en prise de manière opérationnelle avec ledit élément de tige allongé (20) pour y communiquer le mouvement axial sensible à la rotation de ladite partie de déclencheur (18) pivotante autour desdites surfaces de came (26).

4. Instrument chirurgical ultrasonore (10) selon l'une quelconque des revendications précédentes, dans lequel lesdites premières extrémités dudit élément de tige allongé (20) et dudit élément tubulaire allongé (22) comprennent une structure de mise en prise coopérative pour ouvrir et fermer de manière sélective lesdits moyens de lame (21) et de serrage (12) sensibles au mouvement axial relatif entre ledit élément de tige (20) et ledit élément tubulaire (22).

5. Instrument chirurgical ultrasonore (10) selon l'une quelconque des revendications précédentes, dans lequel la lame (21) dudit effecteur comprend une pointe coaxiale sur ledit élément de tige allongé (20).

6. Instrument chirurgical ultrasonore (10) selon la revendication 5, dans lequel ladite lame (21) comprend un arbre tronqué formé de manière solidaire au niveau de la première extrémité dudit élément de tige allongé (20), moyennant quoi ledit élément de tige (20) forme un prolongement de lame ultrasonore.

7. Instrument chirurgical ultrasonore (10) selon la revendication 5, dans lequel ladite lame (21) comprend un arbre tronqué qui est fixé de manière détachable sur la première extrémité dudit élément de tige allongé (20), moyennant quoi ledit élément de tige (20) forme un prolongement de lame ultrasonore.

8. Instrument chirurgical ultrasonore (10) selon la revendication 7, dans lequel ladite lame (21) est fixée sur ledit élément de tige allongé (20) par le biais d'un raccordement fileté à vis (64).

9. Instrument chirurgical ultrasonore (10) selon la revendication 4, dans lequel ladite structure de mise en prise comprend un bras de came monté sur ladite lame (21) pour le mouvement pivotant par rapport à celle-ci.

10. Instrument chirurgical ultrasonore (10) selon l'une quelconque des revendications précédentes, dans lequel ladite partie de poignée (14) comprend des moyens formant bouton-poussoir de verrouillage de déclencheur (70) pour débloquer lesdites parties de déclencheur (16, 18) d'une condition verrouillée sensible à l'enfoncement dudit bouton-poussoir (70) afin de permettre le mouvement relatif entre ledit élément de tige (20) et l'élément tubulaire (22) suite à l'actionnement de ladite partie de déclencheur (18).

11. Instrument chirurgical ultrasonore (10) selon l'une quelconque des revendications précédentes, **caractérisé en ce qu'**il comprend une pluralité de bagues (38) espacées de manière axiale au niveau des noeuds situés le long de la longueur dudit élément de tige allongé (20) et de la lame (21) afin d'empêcher la dispersion des ondes ultrasonores vers ledit élément tubulaire allongé (22) périphérique pendant le fonctionnement dudit instrument.

12. Instrument chirurgical ultrasonore (10) selon l'une quelconque des revendications précédentes, **caractérisé en ce qu'**il comprend des moyens de limitation de rappel (76) sur ledit élément tubulaire allongé (22) afin d'absorber les forces et les tensions de fonctionnement exces-sives générées en réponse à l'actionnement de ladite partie de poignée (14).
